(19) Europäisches Patentamt / European Patent Office / Office européen des brevets

(11) **EP 2 815 693 A1**

(12) **EUROPEAN PATENT APPLICATION**

(43) Date of publication:
24.12.2014 Bulletin 2014/52

(21) Application number: 14173303.0

(22) Date of filing: **20.06.2014**

(51) Int Cl.:
*A61B 1/00* *(2006.01)* *A61B 1/05* *(2006.01)*
*G02B 23/24* *(2006.01)* *A61B 5/06* *(2006.01)*
*H04N 5/225* *(2006.01)* *A61B 19/00* *(2006.01)*

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **21.06.2013 US 201313924350**

(71) Applicant: **OmniVision Technologies, Inc.
Santa Clara, CA 95054 (US)**

(72) Inventor: **Massetti, Dominic
San Jose, CA California 95125 (US)**

(74) Representative: **Kudlek & Grunert Patentanwälte
Postfach 33 04 29
80064 München (DE)**

(54) **Image sensor with integrated orientation indicator background**

(57) An image sensor system for a medical procedure system includes a sensor array for generating image data for a scene and an orientation sensor directly mechanically connected to the image sensor. The orientation sensor generates an electrical signal indicative of orientation of the sensor array. A processor receives the image data and the electrical signal and generates an image of the scene, the image of the scene being altered to compensate for orientation of the sensor array.

EP 2 815 693 A1

**Description**

**BACKGROUND**

**1. Technical Field**

This disclosure is related to image sensors, and, more particularly, to image sensors used in endoscopic imaging.

**2. Discussion of the Related Art**

In the field of minimal access surgery (MAS), cameras or imagers which can include, for example, CMOS image sensors, are typically used for remote diagnosis and precise surgical navigation. Endoscopy generally refers to viewing inside the body for medical reasons using an endoscope, which is an instrument used to examine the interior of a hollow organ or cavity of the body. An endoscope commonly includes a camera or imager used to form an image of the part of the body being examined. Unlike most other medical imaging devices, endoscopes are inserted directly into the organ being examined.
Endoscopy has numerous applications for viewing, diagnosing and treating various parts of the body. For example, colonoscopy refers to the application of endoscopy to view, diagnose and/or treat the large intestine and/or colon. Arthroscopy refers to the application of endoscopy to view, diagnose and/or treat the interior of a joint. Laparoscopy refers to the application of endoscopy to view, diagnose and/or treat the abdominal or pelvic cavity.
The camera attached to the conventional endoscope is used to create an image of the objects or scene within its field of view. The image is displayed with the upright axis of the camera being displayed as the upright axis of the image on the display. Because of the various movements of the endoscope at it is manipulated remotely, or, in the case of a pill endoscope, as it moves freely, the displayed image rotates.
This rotation of the displayed image can complicate the procedure and can adversely affect the outcome of the procedure. A properly oriented stable image would result in faster, more efficient and more successful procedures.

**SUMMARY**

According to one aspect, a medical system for an endoscopic procedure is provided. The system includes an endoscope and a sensor array disposed on the endoscope for generating image data for a scene. An orientation sensor is directly mechanically connected to the image sensor, the orientation sensor generating at least one electrical signal indicative of orientation of the sensor array. A processor receives the image data and the at least one electrical signal and generates an image of the scene, the image of the scene being altered to compensate for orientation of the sensor array.
According to another aspect, an image sensor system is provided. The system includes a sensor array for generating image data for a scene and an orientation sensor directly mechanically connected to the image sensor, the orientation sensor generating at least one electrical signal indicative of orientation of the sensor array. A processor receives the image data and the at least one electrical signal and generates an image of the scene, the image of the scene being altered to compensate for orientation of the sensor array.

**BRIEF DESCRIPTION OF THE DRAWINGS**

The foregoing and other features and advantages will be apparent from the more particular description of preferred embodiments, as illustrated in the accompanying drawings, in which like reference characters refer to the same parts throughout the different views. The drawings are not necessarily to scale, emphasis instead being placed upon illustrating the principles of the inventive concept.

FIG. 1 includes a schematic side view of an endoscope system to which the present disclosure is applicable, according to some exemplary embodiments.
FIG. 2 includes a schematic perspective view of the distal end of a probe of the endoscope system illustrated in FIG. 1, according to some exemplary embodiments.
FIG. 3 includes a detailed schematic cross-sectional diagram of an imaging assembly disposed at a distal end of an endoscopic instrument, according to some exemplary embodiments.
FIG. 4 includes a diagram of a set of mutually orthogonal Cartesian coordinate axes illustrating the functionality of an orientation sensor, e.g., a MEMS accelerometer, used to detect orientation and movement of an image sensor, according to some exemplary embodiments.

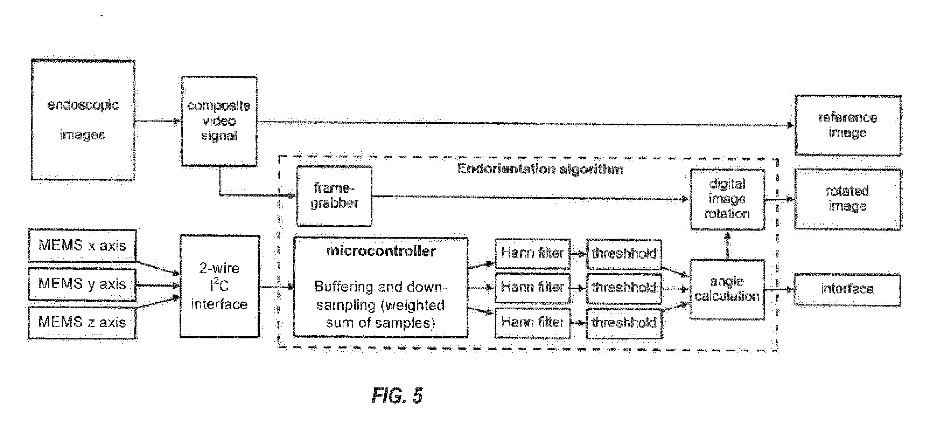

FIG. 5 includes a schematic block diagram of system and method for using data from a three-axis accelerometer to compensate for motion of an endoscopic instrument.

FIG. 6 includes images of a three-axis accelerometer attached to an end of a probe of an endoscopic instrument.

## DETAILED DESCRIPTION

According to exemplary embodiments, the present disclosure describes a system, device and method for providing images from an image sensor located at a distal end of an endoscopic device. The provided image includes compensation for the orientation of the remote image sensor such that the image can be presented on a display with a stable upright axis, i.e., an upright axis which does not rotate with rotation of the image sensor at the remote viewing location. The device to which this disclosure is applicable can be any type of device which provides an image of a remote location from the distal end of a movable device, e.g., an endoscopic surgical device. Such devices to which the present disclosure is applicable can include, for example, colonoscopy devices, arthroscopy devices, laparoscopy devices, angiographic devices, pill endoscopic devices, and any other such remote viewing devices. The present disclosure is applicable to devices used in MAS, including minimally invasive surgery (MIS) and Natural Orifice Translumenal Endoscopic Surgery (NOTES), and other such disciplines. The disclosure is also applicable to any of the devices, systems, procedures and/or methods described in U.S. Application Publication No. US 2012/0086791, published on April 12, 2012, of common ownership. The entire contents of that Application Publication (referred to hereinafter as "the '791 publication") are incorporated herein by reference.

According to some exemplary embodiments, compensation for movement of the remote image sensor is provided by the substantially rigid, mechanical attachment of an orientation sensor to the remote image sensor, such that the orientation sensor is maintained in stationary relationship with the orientation sensor. That is, any movement of the image sensor is also experienced and detected by the orientation sensor. Thus, the orientation sensor detects the movement and orientation of the image sensor and generates one or more electrical signals indicative of the orientation of the image sensor. These orientation signals are received and used by an image processor to generate an image of the remote scene being viewed, with rotational compensation introduced into the image to compensate for any change in orientation, e.g., rotation, of the remote image sensor located, for example, at the distal end of the endoscope.

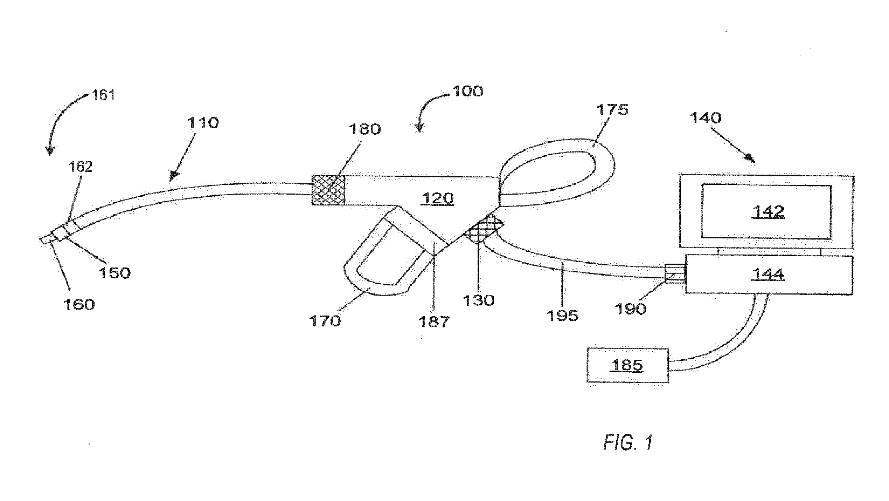

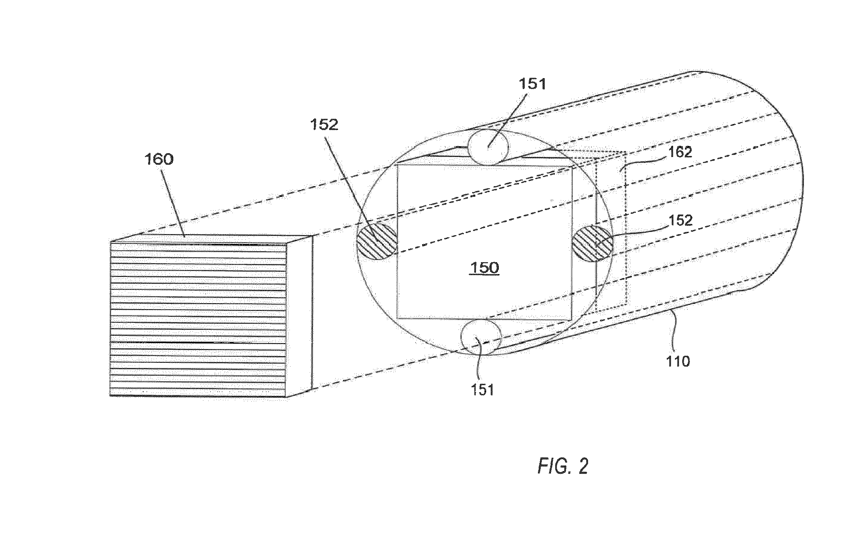

FIG. 1 includes a schematic side view of an endoscope system 100 to which the present disclosure is applicable, according to some exemplary embodiments. FIG. 2 includes a schematic perspective view of the distal end of a probe of endoscope system 100 illustrated in FIG. 1, according to some exemplary embodiments. It will be understood that the system 100 is only one particular exemplary embodiment and that this disclosure is applicable to any type of system using a remote image sensor in which compensation for rotation of the image sensor is desirable. It is also noted that the exemplary embodiment illustrated in FIG. 1 is a modified version of one of the exemplary embodiments described in detail in the '791 publication. As noted above, the present disclosure is also applicable to any of the various devices, systems, procedures and/or methods described in the '791 publication.

Referring to FIGs. 1 and 2, endoscope system 100 includes a probe 110 for insertion into a patient, mounted on a scope core 120, connected to a processing system 130 and ultimately to a monitor/storage station 140 via a cable 195 and a plug 190. The probe 110 includes an image sensor, such as a CMOS image sensor 150, and a lens 160 mounted on a support. As shown in FIG. 2, probe 110 mounts one or more sources of light 151, which can take one of various forms, including an on-probe source such as a light-emitting diode, the end of an optical fiber, other optical waveguide, or other means of transmitting light generated elsewhere in system 100. Probe 110 may also include means for changing the field of view, e.g., swiveling image sensor 150 and/or extending/changing the position of image sensor 150. Probe 110 may take one of various forms, including a rigid structure or a flexible controllable instrument capable of "snaking" down a vessel or other passageway. Probe 110 also supports wires 152 leading from image sensor 150 and light source(s) 151, as well as any additional mechanisms used to control movement of probe 110 and/or image sensor 150 mounted therein.

Lens elements 160 can be movable via a motorized focus control mechanism. Alternatively, lens elements 160 can be fixed in position to give a depth of field providing an in-focus image at all distances from the probe distal end greater than a selected minimum in-focus distance.

Probe 110 connects to a scope core 120, which is a structure that provides a framework to which other components can attach, as well as circuitry for connection of other components. For example, a hand grip handle 170 for an operator can attach to scope core 120. A probe manipulation handle 175 may also attach to scope core 120 and can be used to manipulate probe 110 for movements such as advancement, retraction, rotation, etc. Scope core 120 can include a power source 180 for image sensor 150. Power source 180 can be separate from another power source 185, which can be used for the remainder of system 100. The separation of power sources 180 and 185 can reduce electrical noise. If probe 110 includes a device or means for changing the position of image sensor 150, the controls for that function can be disposed in scope core 120, probe manipulation handle 175, or hand grip handle 170, with keys on the exterior of these components. Power for system 100, apart from image sensor 150, flows either from monitor/storage station 140 or from a separate cell 187 connected to scope core 120 or hand grip handle 170.

When the signal from probe 110 exits the body, or, in non-medical applications, any other viewing site with space and other constraints, it passes through a processing/connector system 130, which, in some exemplary embodiments, is a flexible array of processor circuits that can perform a wide range of functions as desired. The processor circuitry can be organized in one or more integrated circuits and/or connectors between the same, and is housed in one or more modules and/or plugs along the pathway between probe 110 and the point at which the image will be viewed. In some exemplary embodiments, scope core 120 is used as a point of attachment across which a connector system 130 may be mounted. In some exemplary embodiments, as illustrated in FIG. 1, initial processing and analog-to-digital conversion are performed in a connector system module 130 mounted outside scope core 120, possibly to the bottom in order to avoid lengthening scope 100 more than necessary. Connector system module 130 is in turn connected by cable 195 to an end plug 190 attached to monitor/storage station 140, where the image can be viewed.

In other exemplary embodiments, connector system module 130 is connected to the top side of scope core 120 in order to avoid lengthening scope 100 more than necessary. Other exemplary embodiments have more or fewer functions performed in a connector system as described, depending on the preferences and/or needs of the end user. A variety of cables 195 can be used to link the various stages of system 100. For example, one possible link utilizing a Low-Voltage Differential Signaling (LVDS) electrical interface currently used in automotive solutions may allow for up to 10 meters in length, while other options would have shorter reaches. One exemplary embodiment includes connector module 130 placed at the end of cable 195, instead of on scope core 120. Further, in some exemplary embodiments, the final image signal converter integrated circuit chip can be housed in plug 190 designed to link connector system 130 directly to monitor/storage station 140.

In some exemplary embodiments, connector system 130 plugs into monitor/storage station 140, which can include a viewing screen or display 142 and/or a data storage device 144. Standard desktop or laptop computers can serve this function, with appropriate signal conversion being employed to convert the signal into a format capable of receipt by a standard video display device. If desired, monitor/storage station 140 can include additional processing software. In some exemplary embodiments, monitor/storage station 140 is powered by an internal battery or a separate power source 185, as desired. Its power flows upstream to power the parts of system 100 that a not powered by sensor power source 180.

Many alternative embodiments of system 100 can be employed within the scope of the present disclosure. Examples of such alternative embodiments are described in detail in the '791 publication. The embodiment illustrated in FIGs. 1 and 2 is exemplary only.

Continuing to refer to FIGs. 1 and 2, according to the disclosure, in some exemplary embodiments, probe 110 includes an imaging assembly 161 located at its distal end. Imaging assembly 161 includes one or more lens elements 160 and orientation sensor 162 affixed to a back side or proximal side of image sensor 150. In some exemplary embodiments, orientation sensor 162 can be a two-axis or three-axis microelectromechanical system (MEMS) accelerometer. In some particular exemplary embodiments, MEMS accelerometer 162 is stacked directly against and in stationary relation with the back side of integrated circuit image sensor 150. As probe 110 and, therefore, image sensor 150 move, orientation sensor 162 moves with image sensor 150 and tracks its movement and the movement of image sensor 150 over time. Orientation sensor 150 senses inertial changes along two or three axes and provides signals indicative of movement and orientation of image sensor 150 along wires 152 shown in FIG. 2. These signals are used to rotate the image on display 142 such that rotation or other orientation changes of image sensor 150 are compensated and do not result in rotation or other movement of the image on display 142. Orientation sensor or accelerometer 162 can also track its own motion and orientation and, therefore, motion and orientation of image sensor 150, relative to vertical in a standard gravitational field.

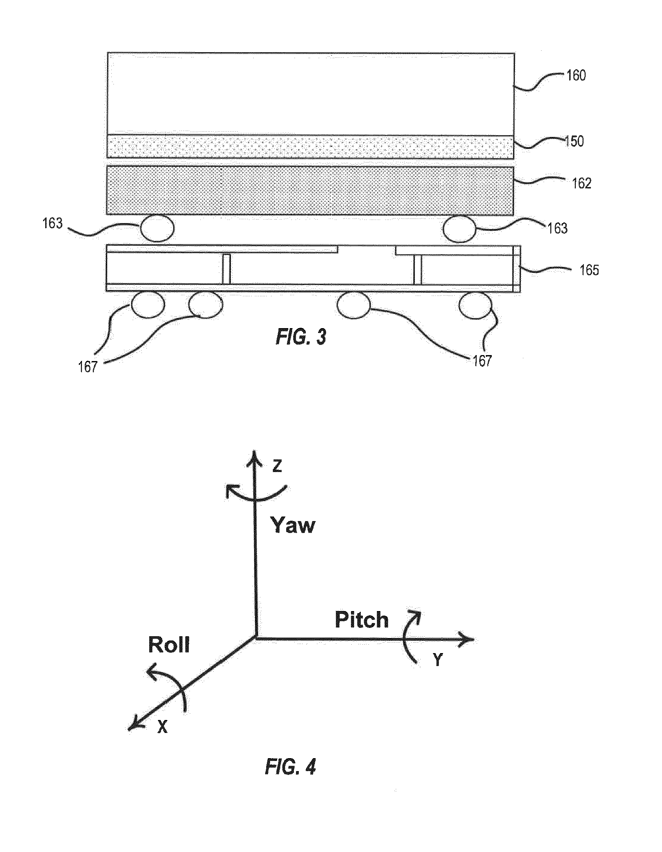

FIG. 3 includes a detailed schematic cross-sectional diagram of imaging assembly 161 disposed at a distal end of an endoscopic instrument, according to some exemplary embodiments. Referring to FIG. 3, imaging assembly 161 includes one or more stacked lens elements 160 disposed over image sensor 150. Lens elements 160 and image sensor 150 are disposed over MEMS accelerometer 162 such that MEMS accelerometer 162 is formed at the back side of image sensor 150. Electrical contact is made to MEMS accelerometer 162 and image sensor 150 via electrical conductors such as solder balls 162, or similar electrical connection construct. The stacked lens elements 160, image sensor 150 and MEMS accelerometer 162 can be electrically connected by solder balls 163 to a wiring construct such as a printed circuit board (PCB) or substrate 165. PCB or substrate 165 includes wiring necessary to conduct the electrical signals for image sensor 150 and MEMS accelerometer to and from image sensor 150 and MEMS accelerometer 162. External connections to PCB or substrate 164 are made via electrical conductors such as solder balls 167, or similar electrical connection construct. In some exemplary embodiments, image sensor 150 and MEMS accelerometer 162 share common electrical connections, such as, for example, power supply connections.

FIG. 4 includes a diagram of a set of mutually orthogonal Cartesian coordinate axes illustrating the functionality of orientation sensor, i.e., MEMS accelerometer 162, used to detect orientation and movement of image sensor 150, according to some exemplary embodiments. Referring to FIG. 4, MEMS accelerometer 162 detects and generates signals indicative of translational or linear motion components along all three mutually orthogonal axes, i.e., the x, y, and z axes. Also, continuing to refer to FIG. 4, MEMS accelerometer 162 detects and generates signals indicative of rotational motion about the three axes, the rotational motions being referred to as pitch, roll and yaw. Hence, MEMS accelerometer 162 detects and generates signals indicative of these six degrees of motion of image sensor 150, thus permitting all motion of image sensor 150 to be compensated for in the presentation of the image on display 142.

According to some exemplary embodiments, MEMS accelerometer 162 can be, for example, a Freescale Xtrinsic MMA8491Q Three-Axis Accelerometer, manufactured and sold by Freescale Semiconductor Inc. of Austin, Texas, USA, or other similar device. MEMS accelerometer 162 senses motion of image sensor 150 in all six degrees of motion and generates electrical motion signals indicative of the detected motion. These motion signals are transmitted along with image data signals from image sensor 150 to processor circuits, such as the processor circuits in processing/connector system 130. These processor circuits generate the image of the scene using both the image data signals and the motion signals to generate the image presented on display 142, with appropriate compensation for the detected motion of image sensor 150. The resulting image maintains a stable orientation on display 142, making the image easier to view by the person conducting the procedure.

According to some exemplary embodiments, exemplary data processing used to generate images for display from data signals generated by image sensor 150 and motion signals generated by orientation sensor 162 with correction/compensation for rotation and other movement of image sensor can be, for example, of the type described in the journal article, "Endoscopic Orientation Correction," by Holler, K., et al., Med Image Comput Comput Assist Interv, 12(Pt 1), 2009, pp. 459-66, the entire contents of which are incorporated herein by reference. Relevant portions of that journal article by Holler, K., et al., are reproduced hereinbelow.

An open problem in endoscopic surgery (especially with flexible endoscopes) is the absence of a stable horizon in endoscopic images. With our "Endorientation" approach image rotation correction, even in non-rigid endoscopic surgery (particularly NOTES), can be realized with a tiny MEMS tri-axial inertial sensor placed on the tip of an endoscope. It measures the impact of gravity on each of the three orthogonal accelerometer axes. After an initial calibration and filtering of these three values the rotation angle is estimated directly. Achievable repetition rate is above the usual endoscopic video frame rate of 30Hz; accuracy is about one degree. The image rotation is performed in real-time by digitally rotating the analog endoscopic video signal. Improvements and benefits have been evaluated in animal studies: Coordination of different instruments and estimation of tissue behavior regarding gravity related deformation and movement was rated to be much more intuitive with a stable horizon on endoscopic images.

## 1. Introduction

In the past years, Natural Orifice Translumenal Endoscopic Surgery (NOTES) has become one of the greatest new challenges within surgical procedures and has the strong potential to eventually succeed minimal invasive surgery (MIS). Currently, MIS interventions are mainly carried out by surgeons using *rigid* laparoscopes inserted in the abdomen from the outside, while gastroenterologists *apply flexible* video-endoscopes for the detection and removal of lesions in the gastro digestive tract (esophagus, stomach, colon, etc.). As the currently practiced *NOTES* and *hybrid* interventions require flexible endoscopes to access the abdominal cavity as well as the surgical instruments and skills to perform the actual intervention, both disciplines and technologies are needed. Gastroenterologists have been trained and accustomed to navigate through the lumen of the colon, stomach or esophagus by pushing, pulling and rotating the flexible video-endoscope, regardless of orientation, rotation and pitch of the endoscope tip inside the patient and the image orientation displayed on the monitor. Surgeons, on the other hand, are used to a fixed relation between the tip of the endoscope and the inside of the patient, as neither one of them is changing their position during the intervention. However, mismatches in the spatial orientation between the visual display space and the physical workspace lead to a reduced surgical performance.

Hence, in order to assist surgeons interpreting and reading images from flexible video-endoscopy, an automated image rectification or re-orientation according to a pre-defined main axis is desirable. The problem of the rotated image is even more important in hybrid NOTES procedures, where an additional micro instrument is inserted through the abdominal wall for exposition and tasks during extremely complex interventions.

In the past, there have been suggested different approaches for motion tracking and image rectification. Several approaches use parameters achieved from registration of intra-operative obtained 3-D data with pre-operative CT or MRI volumes. Such intra-operative 3-D data can be obtained from image-driven approaches like monocular shape-from-shading and structure-from-motion, stereocular triangulation, active illumination with structured light or application of an additional time-of-flight/photonic-mixing-device camera. But even if intra-operative 3-D data can be obtained and reconstructed in real-time, e.g. via time-of-flight cameras needing no data post-processing and having frame rates higher than 30Hz, real-time computation of registration parameters is still a challenge especially since colon or stomach provide less applicable feature points.

Possible tracking technologies include the idea of electro-magnetic tracking, which can be applied to an endoscope. This requires not only an additional sensor in the endoscope's tip but also an external magnetic field. This can easily be disturbed by metallic instruments and leads to several further restrictions. A by far simpler approach to measure the needed orientation angle will be presented in this work and consists of integrating a Micro Electro-Mechanical System (MEMS) based inertial sensor device in the endoscope's tip to measure influencing forces in three orthogonal directions. If the endoscope is not moving, only the acceleration of gravity has an effect on the three axes.

## 2 Method

### 2.1 Technical Approach

To describe the orientation of the endoscope relating to the direction of gravity, an Cartesian "endoscopic board navigation system" with axes *x, y* and *z* (according to the DIN 9300 aeronautical standard) is used as body reference frame. The tip points in *x*-direction which is the boresight, the image bottom is in *z*-direction and the *y*-axis is orthogonal to both in horizontal image direction to the right. Rotations about these axes are called roll $\Phi$ (about *x*), pitch $\Theta$ (about *y*) and yaw $\Psi$ (about *z*). Image rotation has only to be performed about the optical axis *x* which is orthogonal to the image plane. Gravity *g* is considered as an external independent vector. Since there is no explicit angle information, only the impact of gravity on each axis can be used to correct the image orientation. Equation (1) expresses, how rotation parameters $\Phi$, $\Theta$ and $\Psi$ of the IMU (Inertial Measurement Unit) have to be chosen to get back to a corrected spatial orientation with *z* parallel to *g:*

$$\begin{pmatrix} F_x \\ F_y \\ F_z \end{pmatrix} = \begin{pmatrix} 1 & 0 & 0 \\ 0 & \cos(\Phi) & \sin(\Phi) \\ 0 & -\sin(\Phi) & \cos(\Phi) \end{pmatrix} \cdot \begin{pmatrix} \cos(\Theta) & 0 & -\sin(\Theta) \\ 0 & 1 & 0 \\ \sin(\Theta) & 0 & \cos(\Theta) \end{pmatrix} \cdot$$

$$\cdot \begin{pmatrix} \cos(\Psi) & \sin(\Psi) & 0 \\ -\sin(\Psi) & \cos(\Psi) & 0 \\ 0 & 0 & 1 \end{pmatrix} \cdot \begin{pmatrix} 0 \\ 0 \\ g \end{pmatrix} = \begin{pmatrix} -\sin(\Theta)g \\ \sin(\Phi)\cos(\Theta)\,g \\ \cos(\Phi)\cos(\Theta)\,g \end{pmatrix} \qquad (1)$$

with $F_{x,y,z}$ : measured acceleration

Using the two-argument function arctan2 to handle the arctan ambiguity within a range of $\pm\pi$ one finally can compute roll $\Phi$ for $F_x \neq \pm g$ and pitch $\Theta$ for all values:

$$\Phi = \arctan2\left(F_y, F_z\right) \qquad (2)$$

$$\Theta = \arcsin\left(\frac{-F_x}{g}\right) \qquad (3)$$

As *g* determines just 2 degrees of freedom with this approach yaw $\Psi$ cannot be computed. If $F_x \neq \pm g$ ($\rightarrow \Theta = \pm\pi \rightarrow F_y = F_z = 0$) roll $\Phi$ is not determinable either. To avoid movement influence, correction is only applied if superposed acceleration additional to gravity *g* is below boundary value $\Delta F_{absmax}$:

$$\left| \sqrt{F_x^2 + F_y^2 + F_z^2} - g \right| < \Delta F_{absmax} \qquad (4)$$

First, a preceded 3 x 3 calibration matrix, which incorporates misalignment and scaling errors, has to be retrieved by initial measurements. Moreover a peak elimination is the result of down sampling the measuring frequency, which is considerably higher than the image frame rate (up to 400Hz vs. 30Hz). This is realized by summing up separately all *n* sensor values $F_{xi}$, $F_{yi}$ and $F_{zi}$ within an image frame with *i* = 1, ...,*n* and weighting them with a weighting factor $w_i$ with maximal weight $w_0$:

$$w_i = \frac{1}{\frac{1}{w_0} + \left| \sqrt{F_{x_i}^2 + F_{y_i}^2 + F_{z_i}^2} - g \right|} \tag{5}$$

Afterwards the sum has to be normalized by the sum of all weighting factors $w_i$:

$$\begin{pmatrix} F_x \\ F_y \\ F_z \end{pmatrix} = \sum_{i=1}^{n} \left( \begin{pmatrix} F_{x_i} \\ F_{y_i} \\ F_{z_i} \end{pmatrix} \cdot w_i \right) \cdot \sum_{i=1}^{n} (w_i)^{-1} \tag{6}$$

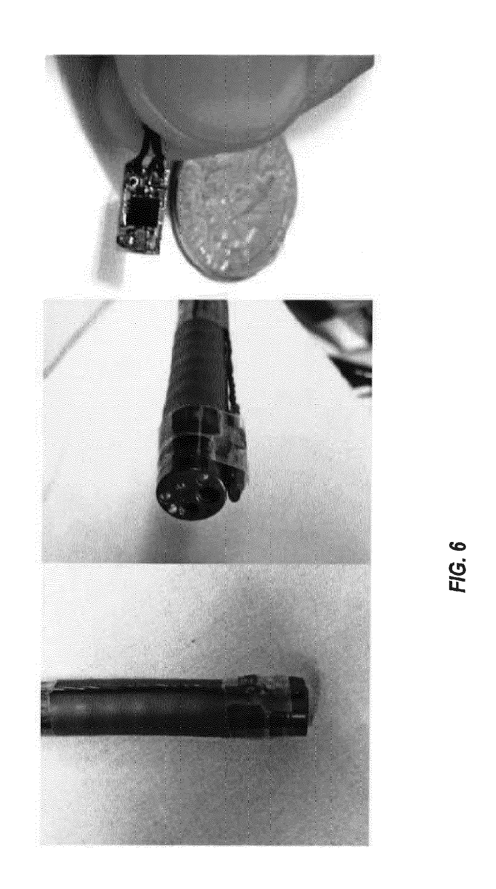

To avoid bouncing or jittering images as a result of the angle correction, additional filtering is necessary. Hence, prior to angle calculation, each axis is filtered with a Hann filter to smooth angle changes and with a minimum variation threshold $\Delta F_{axmin}$ to suppress dithering. As long as superposed acceleration calculated in equation (4) remains below boundary value $\Delta F_{absmax}$, roll $\Phi$ and pitch $\Theta$ can be calculated using equations (2) and (3). Otherwise they are frozen until $\Delta F_{absmax}$ is reached again. If these boundaries are chosen correctly, the results will be continuous and reliable since nearly all superposed movements within usual surgery will not discontinue or distort angle estimation. Both original and rotated image are displayed for security reasons. For potential use with other devices the calculated angle is also transmitted to an external communication interface, as illustrated in FIG. 6.

### 2.2 Image rotation

The measurement data is transferred as a digital signal via a two-wire I2C interface along the flexible endoscope tube. The endoscopic video signal is digitalized via an external USB video capture device with an adequate resolution to provide the usual quality to the operator. By this design the "Endorientation" algorithm is divided into two parts, one part running on a small 8-Bit microcontroller and one parting running as an application on a workstation. Every time the capture device acquires a new frame the software running on the workstation requests the actual acceleration values from the software on the microcontroller. The three acceleration values are used to calculate the rotation angle according to the equations above. The rotation of the frame is performed via the OpenGL library GLUT. The advantage of this concept is the easy handling of time-critical tasks in the software. We can use the sensor sample rate of 400Hz doing some filtering without getting into trouble with the scheduler granularity of the workstation OS. The information of the endoscope tip attitude is available within less than 30ms. Our "Endorientation" approach can be performed in real-time on any off-the-shelf Linux or Windows XP/Vista workstation.

### 2.3 Clinical evaluation

In a porcine animal study, the navigation complexity of a hybrid endoscopic instrument during a NOTES peritoneoscopy with the well-established trans-sigmoidal access was compared with and without Endorientation. The endoscopic inertial measurement unit was fixed on the tip of a flexible endoscope (FIG. 6). Additionally a pulsed DC magnetic tracking sensor was fixed on the hybrid instrument holder for recording the position of the surgeon's hands. To evaluate the benefit of automated MEMS based image rectification, four different needle markers were inserted through the abdominal wall to the upper left and right and the lower left and right quadrants. Under standardized conditions these four needle markers had to be grasped with a trans-abdominal introduced endoscopic needle holder. Displaying alternately originally rotated and automatically rectified images path and duration were recorded and analyzed.

### 3 Results

### 3.1 Technical Accuracy

With the employed sensor there is a uniform quantization of 8 bit for a range of $\pm 2.3$g for each axis. This implies a quantization accuracy of 0.018g per step or 110 steps for the focused range of $\pm$g. This is high enough to achieve a durable accuracy even to a degree within relatively calm movements. This is possible as roll angle $\Phi$ is calculated out of inverse trigonometric values of two orthogonal axes. Single extraordinary disturbed MEMS values are suppressed by

low weighting factors $w_i$. Acceleration occurs only in the short moment of changing movement's velocity or direction. For the special case of acceleration with the same order of magnitude as gravity, $\Delta F_{absmax}$ can be chosen small enough to suppress calculation and freeze the angle for this short period of time. By choosing a longer delay line for the smoothing Hann filter and a higher minimum variation threshold $\Delta F_{axmin}$, correction may be delayed by fractions of a second but will be stable even during fast movements.

### 3.2 Clinical Evaluation

In the performed experiments, it could clearly be shown that grasping a needle marker with an automatically rectified image is much easier and therefore faster than with the originally rotated endoscopic view. In comparison to the procedure without rectification the movements are significantly more accurate with by factor 2 shorter paths and nearly half the duration. The two parameters *duration* and *path length* are strongly correlated and can be regarded as a significant measure for the complexity of surgical procedures. Since both are decreased with the application of image rectification, the complexity of the complete procedure can be reduced.

### 4 Discussion

As described in the previous section, an automatic rectification (or re-orientation) of the acquired endoscopic images in real-time assists the viewer in interpreting the rotated pictures obtained from a flexible videoscope. This is especially important for physicians, who are used to naturally rectified endoscopic images related to a patient-oriented Cartesian coordinate system within their surgical site. In contrast, gastroenterologists have learned by combination of long experience, anatomical knowledge and spatial sense how to use and interpret an endoscope-centered (tube-like) coordinate system during their exploration of lumenal structures, even if the displayed images are rotating. Our described experiments included surgeons originally unrelated to flexible endoscopes. For future research, we will also include gastroenterologists, who are experienced reading and interpreting rotated and non-rectified image sequences. Possibly, in the future of NOTES, dual monitor systems will be needed to support both specialists during the intervention.

### Combinations of Features

Various features of the present disclosure have been described above in detail. The disclosure covers any and all combinations of any number of the features described herein, unless the description specifically excludes a combination of features. The following examples illustrate some of the combinations of features contemplated and disclosed herein in accordance with this disclosure.

In any of the embodiments described in detail and/or claimed herein, the processor can rotate the image to compensate for the orientation of the sensor array.

In any of the embodiments described in detail and/or claimed herein, the orientation sensor can be a two-dimensional orientation sensor.

In any of the embodiments described in detail and/or claimed herein, the orientation sensor can be a three-dimensional orientation sensor.

In any of the embodiments described in detail and/or claimed herein, the orientation sensor can be an accelerometer.

In any of the embodiments described in detail and/or claimed herein, the accelerometer can be a two-axis accelerometer.

In any of the embodiments described in detail and/or claimed herein, the accelerometer can be a three-axis accelerometer.

In any of the embodiments described in detail and/or claimed herein, the accelerometer can be a micro-electro-mechanical systems (MEMS) accelerometer.

In any of the embodiments described in detail and/or claimed herein, the sensor array can be an integrated circuit having a first side and a second side, and the MEMS accelerometer can be mounted on the second side of the sensor array integrated circuit.

In any of the embodiments described in detail and/or claimed herein, the system can further comprise a display for displaying the image of the scene.

In any of the embodiments described in detail and/or claimed herein, the image sensor and the orientation sensor can be positioned in contact with each other in a stacked configuration.

In any of the embodiments described in detail and/or claimed herein, the image sensor and the orientation sensor can be electrically connected together.

In any of the embodiments described in detail and/or claimed herein, the image sensor and the orientation sensor can share common electrical conductors.

In any of the embodiments described in detail and/or claimed herein, the sensor array and the orientation sensor can be mounted in an endoscopic medical instrument.

While the present inventive concept has been particularly shown and described with reference to exemplary embodiments thereof, it will be understood by those of ordinary skill in the art that various changes in form and details may be made therein without departing from the spirit and scope of the present inventive concept as defined by the following claims.

**Claims**

1. A medical system for an endoscopic procedure, comprising:

   an endoscope;
   a sensor array disposed on the endoscope for generating image data for a scene; and
   an orientation sensor directly mechanically connected to the image sensor, the orientation sensor generating at least one electrical signal indicative of orientation of the sensor array; and
   a processor for receiving the image data and the at least one electrical signal and generating an image of the scene, the image of the scene being altered to compensate for orientation of the sensor array.

2. An image sensor system, comprising:

   a sensor array for generating image data for a scene; and
   an orientation sensor directly mechanically connected to the image sensor, the orientation sensor generating at least one electrical signal indicative of orientation of the sensor array; and
   a processor for receiving the image data and the at least one electrical signal and generating an image of the scene, the image of the scene being altered to compensate for orientation of the sensor array.

3. The medical system of claim 1 or image sensor system of claim 2, wherein the processor rotates the image to compensate for the orientation of the sensor array.

4. The medical system or image sensor system of any of the preceding claims, wherein the orientation sensor is a two-dimensional orientation sensor, or wherein the orientation sensor is a three-dimensional orientation sensor.

5. The medical system or image sensor system of any one of the preceding claims, wherein the orientation sensor is an accelerometer, especially a two-axis accelerometer or a three-axis accelerometer.

6. The medical system or image sensor system of claim 4, wherein the accelerometer is a micro-electro-mechanical systems (MEMS) accelerometer.

7. The medical system or image sensor system of claim 6, wherein:

   the sensor array is an integrated circuit having a first side and a second side; and
   the MEMS accelerometer is mounted on the second side of the sensor array integrated circuit.

8. The medical system or image sensor system of any one of the preceding claims, further comprising a display for displaying the image of the scene.

9. The medical system or image sensor system of any one of the preceding claims, wherein the image sensor and the orientation sensor are positioned in contact with each other in a stacked configuration.

10. The medical system or image sensor system of any one of the preceding claims, wherein the image sensor and the orientation sensor are electrically connected together.

11. The medical system or image sensor system of any one of the preceding claims, wherein the image sensor and the orientation sensor share common electrical conductors.

12. The image sensor system of any of claims 2 to 11, wherein the sensor array and the orientation sensor are mounted in an endoscopic medical instrument.

*FIG. 1*

*FIG. 2*

EP 2 815 693 A1

FIG. 3

FIG. 4

FIG. 5

*FIG. 6*

Europäisches
Patentamt
European
Patent Office
Office européen
des brevets

# EUROPEAN SEARCH REPORT

**Application Number**

EP 14 17 3303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2009 013761 A1 (FRAUNHOFER GES FORSCHUNG [DE]; UNIV MUENCHEN TECH [DE]) 23 September 2010 (2010-09-23) * paragraphs [0012] - [0037], [0048] - [0050]; claims 1-3; figures 1-3 * | 1-12 | INV. A61B1/00 A61B1/05 G02B23/24 A61B5/06 |
| X | JP 2006 042900 A (OLYMPUS CORP) 16 February 2006 (2006-02-16) * paragraphs [0053] - [0055], [0069] - [0072]; figure 20 * | 1-12 | ADD. H04N5/225 A61B19/00 |
| X | JP 2010 008483 A (NISCO KK) 14 January 2010 (2010-01-14) * paragraphs [0019] - [0021], [0028], [0030] - [0033]; claims 1,2,4,6; figures 1,10-15 * | 1-12 | |
| X | US 6 471 637 B1 (CHATENEVER DAVID [CA] ET AL) 29 October 2002 (2002-10-29) * column 3, line 2 - line 24 * * column 4, line 22 - column 7, line 14 * * column 8, line 60 - column 9, line 18 * * column 10, line 5 - column 11, line 29; figures 1-4,8-11 * | 1-5,8, 10-12 | |
| X | US 5 899 851 A (KONINCKX ROBERT PHILIPPE [BE]) 4 May 1999 (1999-05-04) * column 1, line 66 - column 2, line 61; claims 1,3,9,10 * | 1,2,5, 8-12 | TECHNICAL FIELDS SEARCHED (IPC) A61B G02B H04N |
| A | US 2012/157769 A1 (ZHU PENG FEI [CN] ET AL) 21 June 2012 (2012-06-21) * paragraphs [0021] - [0026]; figure 4 * | 4-7 | |

-/--

The present search report has been drawn up for all claims

| Place of search | Date of completion of the search | Examiner |
|---|---|---|
| Munich | 14 November 2014 | Rick, Kai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**Europäisches Patentamt**
**European Patent Office**
**Office européen des brevets**

# EUROPEAN SEARCH REPORT

Application Number

EP 14 17 3303

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | KURT HÖLLER ET AL: "Endoscopic Orientation Correction", 20 September 2009 (2009-09-20), MEDICAL IMAGE COMPUTING AND COMPUTER-ASSISTED INTERVENTION Â MICCAI 2009, SPRINGER BERLIN HEIDELBERG, BERLIN, HEIDELBERG, PAGE(S) 459 - 466, XP019130352, ISBN: 978-3-642-04267-6 * the whole document * | 1-12 | |

TECHNICAL FIELDS
SEARCHED (IPC)

The present search report has been drawn up for all claims

| Place of search | Date of completion of the search | Examiner |
|---|---|---|
| Munich | 14 November 2014 | Rick, Kai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 17 3303

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2014

| Patent document cited in search report | | Publication date | Patent family member(s) | | | Publication date |
|---|---|---|---|---|---|---|
| DE 102009013761 | A1 | 23-09-2010 | AT | 550982 | T | 15-04-2012 |
| | | | DE | 102009013761 | A1 | 23-09-2010 |
| | | | EP | 2323541 | A1 | 25-05-2011 |
| | | | ES | 2384949 | T3 | 16-07-2012 |
| | | | WO | 2010105946 | A1 | 23-09-2010 |
| JP 2006042900 | A | 16-02-2006 | JP | 4526320 | B2 | 18-08-2010 |
| | | | JP | 2006042900 | A | 16-02-2006 |
| JP 2010008483 | A | 14-01-2010 | NONE | | | |
| US 6471637 | B1 | 29-10-2002 | NONE | | | |
| US 5899851 | A | 04-05-1999 | AU | 6991794 | A | 06-02-1995 |
| | | | EP | 0712289 | A1 | 22-05-1996 |
| | | | JP | 3476195 | B2 | 10-12-2003 |
| | | | JP | H08512223 | A | 24-12-1996 |
| | | | NL | 9301210 | A | 01-02-1995 |
| | | | US | 5899851 | A | 04-05-1999 |
| | | | WO | 9501749 | A1 | 19-01-1995 |
| US 2012157769 | A1 | 21-06-2012 | CN | 102525386 | A | 04-07-2012 |
| | | | US | 2012157769 | A1 | 21-06-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20120086791 A

**Non-patent literature cited in the description**

• **HOLLER, K. et al.** Endoscopic Orientation Correction. *Med Image Comput Comput Assist Interv,* 2009, vol. 12, 459-66